# EUROPEAN PATENT APPLICATION

(11) **EP 2 677 321 A1**
(43) Date of publication of application: **25.12.2013**
(21) Application number: 12172801.8
(22) Date of filing: 20.06.2012
(51) Int. Cl.: G01N 35/00, C12Q 1/00

(54) **Integrated device for nucleic acid hybridizations and immuno-assays**

(71) Applicant: Innogenetics N.V., 9052 Zwijnaarde (BE)
(72) Inventor: The designation of the inventor has not yet been filed
(74) Representative: BiiP cvba

(57) **Abstract**

The present invention is related to automated and semi-automated nucleic acid hybridizations and immuno-assays, and methods for performing nucleic acid hybridizations or immuno-assays on an integrated device. Specifically, this invention is an integrated device and method for performing solid support based nucleic acid hybridizations and immuno-assays.

## Description

### Field of the invention

The present invention is related to automated and semi-automated nucleic acid hybridizations and immuno-assays and methods for performing nucleic acid hybridizations or immuno-assays on an integrated device. Specifically, this invention is an integrated device and method for performing solid support based nucleic acid hybridizations and immuno-assays.

### Background of the invention

In clinical testing emphasis is often on identification or quantification of nucleic acids and proteins present in a sample of a patient by probe - target nucleic acid hybridizations and antibody - antigen immuno-assays, respectively. Clinical laboratories increasingly rely upon automated assay equipment in order to handle large numbers of assays efficiently in terms of time and cost, and further, to increase the reliability of such assays by decreasing the amount of human intervention involved in such assays. This reduction in human intervention necessitates a corresponding increase in equipment and devices which ensure the accurate performance of such automated assays.
In the past decades, many high throughput automation devices have been introduced. Typical examples are described in e.g. US patent 5,698,450; US patent applications 2008/0318323, 2011/0262896, 2011/0275058, 2002/0098117; and WO2004/004905. All these devices are technically very complex and expensive. The dispensing, agitating and heating of reagents and other assay solutions, and the controlled pre-incubation, incubation, washing, and detection of the target of interest in the patient sample can, with such automated devices, still be problematic. It is of vital importance that the patient samples be handled in a manner which prevents their contamination and mishandling of reagents in the assay protocol, respectively.
Also, analyses of a limited number of patient samples on a high throughput automated apparatus are cost-ineffective. The analyses of only a few patient samples, e.g. in the laboratory of a small hospital or a medical home practice, are very time consuming, are often run consecutively, require trained personnel to perform precise measurements, etc. Each step of these analyses typically needs to be repeated several times, each time with inclusion of proper controls, to acquire statistically significant data. The processes are often wasteful of costly reagents, prone to human mistakes, require numerous man-hours, and are generally slow.
Nucleic acid hybridizations demand precise temperature control. Rates of hybridization and equilibrium concentrations of nucleic acid double strands depend strongly on temperature and therefore accurate comparisons between hybridization experiments require that the experiments be run at the same temperature. In addition, precise temperature programming during a diagnostic test is critical to reduce background binding.

There is thus a need for a device that can reduce the extent of manual manipulation during nucleic acid hybridizations and immuno-assays, which in addition minimizes reagent waste, and allows precise temperature control.

### Summary of the invention

According to one embodiment of the invention, an integrated device is provided for processing multiple biological samples, each sample comprising multiple nucleic acids, proteins or other target molecules or species of molecules, comprising the step of dispensing each reagent to effect the binding of nucleic acids, proteins or other target molecules onto all selected solid supports simultaneously.
According to another embodiment, an integrated device is provided for processing multiple biological samples, each sample comprising multiple nucleic acids, proteins or other target molecules or species of molecules, comprising the step of displacement of only the minimal volume of each reagent required per step of the assay for the analysis of each said sample.
According to another embodiment, an integrated device is provided for processing multiple biological samples each sample comprising multiple nucleic acids, proteins or other target molecules or species of molecules, comprising an integrated heating block for simultaneous heating of assay solutions in reagent containers and the reaction trays.

### Brief description of the figures

Figure 1 is a perspective view of one embodiment of an integrated diagnostic device according to the present invention.
Figure 2 is a perspective view of the assay platform of the integrated diagnostic device of Figure 1 and according to the present invention.
Figure 3 is a cross-sectional view along the line A-A of the assay platform in Figure 2 of the integrated diagnostic device of Figure 1 and according to the present invention.
Figure 4 a and b is a transverse and longitudinal section, respectively, of the temperature controllable tray comprising reagent containers and reaction trays of the integrated diagnostic device of Figure 1 and according to the present invention.
Figure 5 is a perspective view of the reaction trays of the integrated diagnostic device of Figure 1 and according to the present invention.
Figure 6 schematically shows a dispensing system according to an embodiment of the present invention.
Figure 7 schematically shows an aspirating system according to an embodiment of the present invention.

### Description of the invention

The invention is an integrated device that can be used to carry out nucleic acid hybridizations and immuno-assays in an automated, accurate, safe, economic and user friendly manner. The integrated device comprises an assay platform consisting of a dispense head; reagent containers; a waste container; and a tray comprising a heating means, reagent containers, reaction trays, and an agitation means. The present invention provides an integrated device capable of precise thermal and fluid control.

The embodiments disclosed herein relate to systems and methods for reducing the amount of manual manipulations required for solid support based nucleic acid hybridizations and immuno-assays used in molecular biology, while preserving reagents, minimizing waste, and controlling temperature and assay solution transport.

The device may be used in an assay for detecting, e.g. specific nucleic acids or antigens in a sample. Furthermore, the device may be used in an assay for detecting, e.g. aberrations in nucleic acids and proteins and derivatives thereof, and other biochemical compounds present in a bodily sample. Furthermore, the device may be used in multiparameter assays investigating different nucleic acids and/or different immunological properties simultaneously for a given sample. For nucleic acid based applications, typical examples are genotyping assays and drug resistance assays for viral and bacterial pathogens. Multiparameter panels detecting the presence of nucleic acids of disease-causing agents in infectious diseases like e.g. gastro-intestinal panel, sepsis panel, meningitis panel, food-pathogen panel, etc. can be run in the device. Other applications looking at different polymorphisms or mutations in a single or in different genes simultaneously are used often in genetic testing. Examples are e.g. mutation testing for cystic fibrosis, HLA typing for transplantation or disease association studies, SNP polymorphisms in pharmacogenetics, etc.
For immunological applications, typical examples are confirmatory assays used in infectious disease testing. The device may be used for examining the presence of antibodies against different antigens simultaneously to confirm and discriminate different viruses in a clinical sample, e.g. HTLV I and II or HIV-1 and HIV-2. Other applications which require investigation of antibodies against different antigens simultaneously may be for diagnosis of auto-immune diseases. Yet other assays make use of sub-variants of the same antigen which are being detected by different antibodies.

The integrated device according to the invention can be used for processing multiple biological samples, each said sample comprising multiple nucleic acids, proteins or other target molecules or species of molecules, comprising the step of displacement of one unit of assay solutions required per step of the assay for analysis of each said sample. According to one embodiment, said device comprises a dispensing head, preferably comprising a multiple channel dosing chamber, whose channels can preferably be separately operable. According to another embodiment said device further comprises an integrated heating block, likely a metal block, wherein said metal is preferably aluminium.
According to another embodiment the device further comprises a tray with reagent vessels and reaction vessels, wherein the reaction vessels can partially be covered with a lid. The device may also comprise an edge pivoting means.
The integrated device will further comprise selected fixed solid supports that will preferably be possible to process in parallel simultaneously. In one embodiment of the device the solid support is a strip, preferably a nitrocellulose, nylon, or a polyvinylidene fluoride PVDF strip. According to another embodiment, the solid support is a reaction vessel, preferably positioned vertically, or multiple units of reaction vessels.

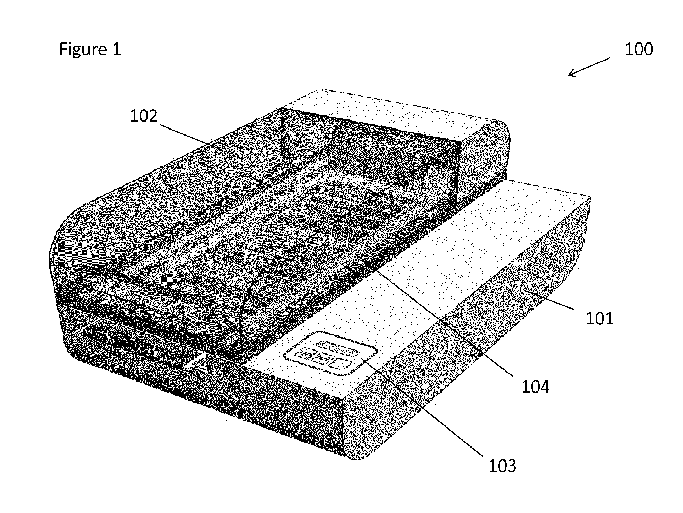

Figure 1 shows a perspective view of one embodiment of an integrated device 100 that can be used to perform nucleic acid hybridizations and immuno-assays with solid supports. The device 100 shown in Figure 1 includes a housing 101, a cover 102, a control panel 103, and an assay platform 104. The number of assay platforms 104 can vary to any number, and is preferably 1, preferably 2, or preferably ranging from 1 to 4. Each assay platform can operate independently. In a preferred embodiment the integrated device 100 according to this invention runs stand-alone when performing assays. The device can be programmed in desirable embodiments to perform a variety of dispensing, heating, agitating and incubating steps with each step having a preselected period of time within which to occur. The control logic can be modified to accommodate other assays or other procedures.
Applications on a PC or USB or memory card or the like are required for service software, manufacturing tools, e.g. calibrations and tests, protocol development or protocol download. In an embodiment the control panel 103 is a LCD display and keypad, wherein the LCD display can be standard 7-segment, alpha numeric, or custom designed; the keypad is a membrane style keypad with approximately 6 to 10 keys; and holds indicator and status LEDs, e.g. a power indicator, alarm, and in progress signal.

The following approximate physical specifications are used with desirable embodiments of the device. The overall length is 50 to 80 cm, desirably 63 cm. The overall width is 30 to 80 cm, desirably 38 cm. The overall height is 15 to 40 cm, desirably 24 cm. The total weight is desirably less than 20 kg. These dimensions provide a commercially desirable device that can be readily used in most laboratories for performing automated solid support based nucleic acid hybridizations and immuno-assays.

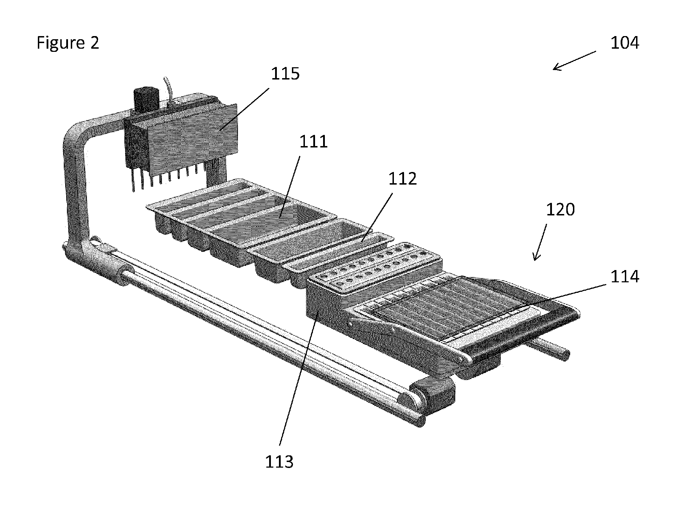

Figure 2 shows a perspective view of one embodiment of the assay platform 104 that can be used to perform nucleic acid hybridizations and immuno-assays with solid supports according to this invention. The assay platform 104 includes a dispense head 115, reagent containers 111, a waste container 112, and a tray 120 composed of reagent containers 113 and reaction trays 114.

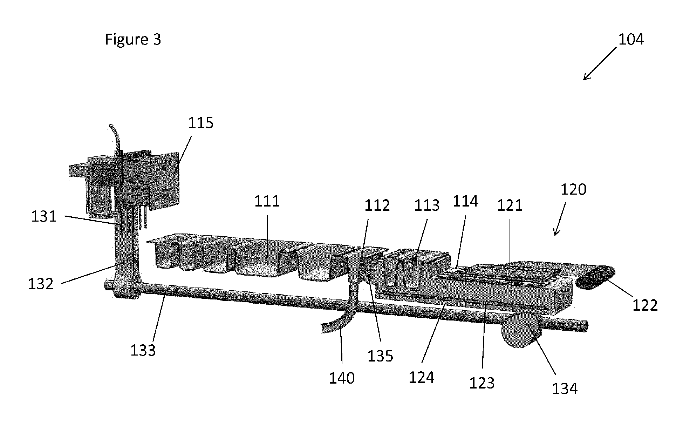

Figure 3 is a cross-sectional view of one embodiment of the assay platform 104 showing a dispense head 115, reagent containers 111, a waste container 112, and a tray 120 composed of reagent containers 113 and reaction trays 114. The reaction trays 114 can be covered with a cover lid 121 by a handle bar 122. In a preferred embodiment the tray 120 contains a heating means 123 and insulating means 124. In an embodiment according to the present invention agitation of reagents is accomplished with an edge pivoting rocking mechanism 134 and 135. In an embodiment the dispense head 115 is mounted on a dispense head holder 132 and can move along a rail 133 in X direction for parallel transfer of assay reagents to and from the reaction trays 114.

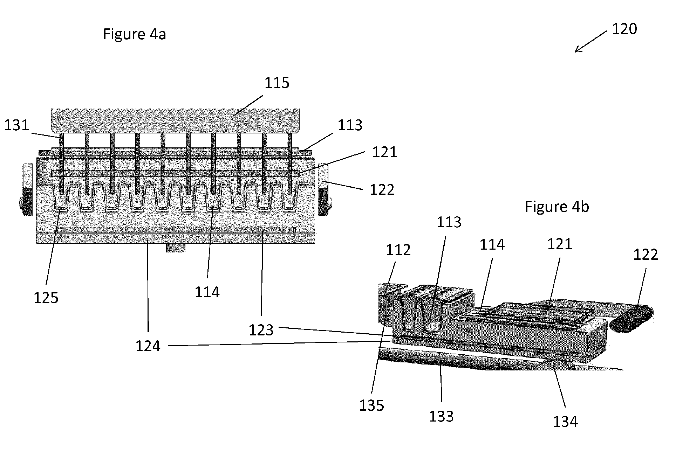

Figure 4 a and b is a transverse and longitudinal section, respectively, of tray 120 comprising components as disclosed herein above annotated with the same numerical annotations. In a preferred embodiment the reaction tray has a cavity 125 for cooling air.

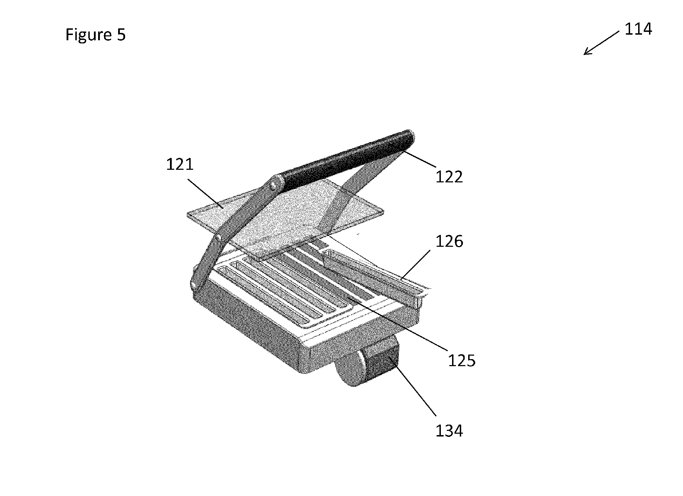

Figure 5 is a perspective view of the reaction trays 114. In a preferred embodiment a reaction vessel 126 is placed in a reaction tray. Also, during processing, the cover lid 121 presses the reaction vessels 126 against the reaction trays 114 that are in direct contact with the heating means 123.

The reaction vessel unit can be a single reaction vessel or the reaction vessel unit can consist of any number of reaction vessels up to the maximum number of reaction vessels that can be hold by the reaction tray 114. In a preferred embodiment the reaction vessel unit contains 1 to 10 reaction vessels, preferably 1 reaction vessel, preferably 10 reaction vessels.

Each reaction vessel 126 holds a reactant immobilized on a solid support. The support may be fabricated out of any material suitable for immobilizing the reaction component, e.g., polyethylene, nitrocellulose, nylon, polyvinylidene fluoride (PVDF), glass, magnetic beads etc. The support may also be of any desired size or form which can be accommodated by the reaction vessel 126 or reaction tray 114 of tray 120. In a preferred embodiment of this invention strips are preferred. In a preferred immunoassay, the reactant will usually be an antigen or antibody. In a preferred nucleic acid hybridization assay, the reactant will usually be DNA, RNA, or modifications thereof, such as said nucleic acids having incorporated therein chemical modifications for improvement of nucleic acid duplex formation and stability, e.g. 2-O-methyl groups.

The immobilized reactant may be a component of a sample, or it may be a reagent which acts upon the sample or upon another reagent (e.g., a secondary antibody which binds a primary antibody, or complementary nucleic acids). The reaction component may be reversibly or irreversibly immobilized on the support by any art-recognized technique. It may be bound covalently, or noncovalently, and directly or indirectly.

The solid support means is sequentially exposed to other reaction components, such as samples, reagents, and washing solutions. At the end of the assay sequence, the assay results are determined.

The samples of the present invention may be biological fluids such as blood, plasma, sera, CSF, saliva, sputum, tears, urine, milk and the like, or tissues which have been solubilized for assay purposes, or standard samples, e.g. consisting of protein materials. The reagents may be antibodies (or other binding proteins), antigens (or other target molecules), nucleic acids, enzymes, enzyme substrates, buffers, chromogens, small molecular inhibitors, fluorophores, and the like.

In some embodiments, there are 10 reaction vessels 126 in a tray 120. Optionally, 8 reaction vessels 126 are for analyses of samples and one each for a positive and a negative control. Alternatively, proper controls are added onto a solid support designed for sample analysis. In this event, preferably, none of the reaction vessels 126 are used for control purposes only.

In a preferred embodiment multiple solid supports can be processed in parallel on a single assay platform.

The means to permit rocking can be a pivoting connection, such as an axle or hinge. In an embodiment the axis of rotation is on an end of the tray 120, said end of the tray remains essentially fixed and the other end moves up and down.
The rocking mechanism of this invention is desirably provided by a "step motor" 134. The step motor 134 rotates the tray 120 both clockwise and counterclockwise about an axis of rotation. The axis of rotation of the tray 120 and rail 133 for movement of the dispense head 115 are perpendicular but not necessarily in the same plane.
In some embodiments, the tray 120 is pivoted about an axis of rotation, with the reaction trays 114 being furthest from the axis of rotation. The tray 120 is gently rocked to enable the reagents, sample or wash solution to spread evenly over the solid support means and mix uniformly with any soluble assay components already in the reaction vessels 126.

In a preferred embodiment an assay solution is removed from the reaction vessel 126 by rotating the tray 120 to a positive angle of rotation for the tray 120 and aspirating the solution from the reaction vessel 126 by the dispense head 115. The aspiration position of the tray 120 is desirably held for a time sufficient to aspirate the assay solution completely.
In a preferred embodiment, the tray 120 is tilted up to an inclination enabling efficient aspiration of the solutions away from the solid support means.

In a preferred embodiment the number of reagent containers 111 equals the number of assay solutions to be kept at room temperature and required to run the assay of choice. This number can vary between 1 and 10, preferably between 3 and 7, and is 5 in a preferred embodiment. In some embodiments the reagent containers 111 can be disposable and configured for single use. In some embodiments the reagent containers 111 can be made of reusable material. The assay solutions can be wash solutions, buffers, distilled water, and the like needed to run the assay of choice, and can be supplied as a kit, optionally in prefilled disposable reagent containers.

The following operating specifications are used with desirable embodiments of the device and method of this invention. Input voltage for the apparatus can be 110 or 220 volts. Operating temperatures range from about 4 to about 99 degrees Celsius, preferably from ambient temperature to about degrees Celsius. A preferred ambient temperature can be room temperature or even less when the device is placed e.g. in a refrigerated environment.

In a preferred embodiment the reaction vessels 126 are held in place in the reaction trays 114 by a cover lid 121 that includes a handle bar 122 for opening and closing the reaction trays 114. The cover lid 121 avoids evaporation of assay solutions in the reaction vessels 126 and limits contamination of a reaction vessel 126 by assay solutions or patient sample from a neighbouring reaction vessel 126. Also, during processing, the cover lid 121 presses the reaction vessels 126 against the reaction trays 114 that are in direct contact with the heating means 123.
In a preferred embodiment the cover lid 121 partially covers the reaction vessels 126 leaving at least one opening to add or remove assay solutions or a patient sample in each reaction vessel 126 according to the assay protocol of choice. Furthermore, there are two openings, one at each end of the reaction vessel 126, the one closest to the reagent containers 113 for dispensing and aspirating assay solutions and the second one for adding (patient) sample in the reaction vessel 126. The cover lid 121 is constructed from a high temperature plastic to prevent sagging or softening at the higher operating temperatures of the device 100. A suitable plastic may include polysulfone. Polysulfone possesses the requisite temperature characteristics and is transparent, which allows direct viewing of the solid support means during processing.

In some embodiments the reaction vessels 126 can be disposable and configured for single use. In some embodiments the reaction vessels 126 can be made of reusable material. The reaction vessel 126 can be preloaded with a solid support according to the assay of choice. In yet another embodiment, the reaction vessel 126 is the solid support itself. The reaction vessel 126 may also be of any desired size or form which can be accommodated by the reaction tray 114 of tray 120. A preferred form is cubical or cylindrical. The reaction vessel 126 may be placed in any direction, preferably horizontal or vertical. In a preferred embodiment the reaction vessel 126 is cubical and placed horizontally. In some other embodiments the reaction vessel 126 is cylindrical and placed vertical. The reaction vessel 126 can be supplied as part of a kit.

In a preferred embodiment transport of assay solutions is handled by the dispense head 115. The dispense head 115 consists of individual dosing chambers. The number of dosing chambers is equal to the number of reaction trays 114. A step motor advances the dispense head 115 along a rail 133. The step motor moves the dispense head by means of a cord, chain, belt or equivalent flexible device. In a preferred embodiment the dispense head moves in a direction for dispensing, aspirating, and optionally mixing assay solutions. Movements in this direction can be accomplished by the same movement means as for x direction movement of the dispense head. In a preferred embodiment movement of the dispense head is fully automated and robotic.

In a preferred embodiment the integrated device according to the present invention is free of conduits for handling of assay solutions. Consequently, there is no need for preloading or priming with assay solutions as state of the art devices do. This saves a lot on assay solutions, especially those that are expensive, and enables full use of tests assays delivered in a diagnostic kit, e.g. a kit for 10 tests contains assay solutions to run 10 test without the need to add extra volume of assay solutions to compensate for waste of said solutions by preloading or priming liquid conduits in the device.

Assay solutions such as buffers and wash solutions to be used at ambient temperature are desirably placed in reagent containers 111. Other assay solutions to be used at elevated temperatures are desirably placed in reagent containers 113. The volume of assay solutions dispensed to the reaction vessels 126 is less than 0.5 ml, 0.5 ml, 1 ml, 1.5 ml, 2 ml, 2.5 ml, 3 ml, more than 3 ml. In a preferred embodiment the volume is desirably 1 ml and desirably a multitude of 1 ml for a reaction vessel 126 having a preferred solid support section of approximately 0.32 cm by 10 cm. The aggregate volume of assay solutions dispensed depends on the number of samples being assayed. A desirable reagent container volume is 180 ml for washing buffer and 30 ml for reagent solutions. The approximate volume of reagent solutions required for a complete assay of a full tray 120 is desirably about 180 ml of wash buffer and about 30 ml of each reagent solution.

In some embodiments, the waste container 112 includes a drain feature 140, which allows for removal of assay solutions from the assay platform 104. In some embodiments, the drain feature 140 is a plastic conduit and is connected to a waste bottle 141. In some embodiments, the conduit or the waste bottle is coupled to a pump, such as a peristaltic pump, a piston pump, a hydraulic pump, a vacuum pump or the like.

Also provided is a temperature control device configured to control the temperature of the assay solutions in the tray of the assay platform.
In a preferred embodiment the heating means 123 includes a thermal plate that is designed and constructed to maximize heat transfer between Peltier devices and the reagent containers 113 and reaction trays 114, all part of tray 120. The thermal plate is designed to provide rapid temperature response and uniform temperature distribution across the tray 120 in general, and the assay solutions in reagent containers 113 and in the reaction vessels 126 in particular. The thermal plate has minimal thermal mass and a high degree of flatness to maximize thermal contact area.
The heating means 123 is typically capable of temperature accuracies of about 0.5° C. A heating means 123 provides temperatures from ambient temperature to 100 degrees Celsius. Preferably, the heating block is manufactured from a solid piece of metallic material of high thermal conductivity and, most preferably, it is casted from an aluminium or copper alloy.

In some embodiments, the heated reagent container 113 can have a lid for insulating said container and avoiding evaporation of assay solutions dispensed herein. Each lid can have entry ports, each configured to allow entry of the dosing needles 131 of the dispense head 115.

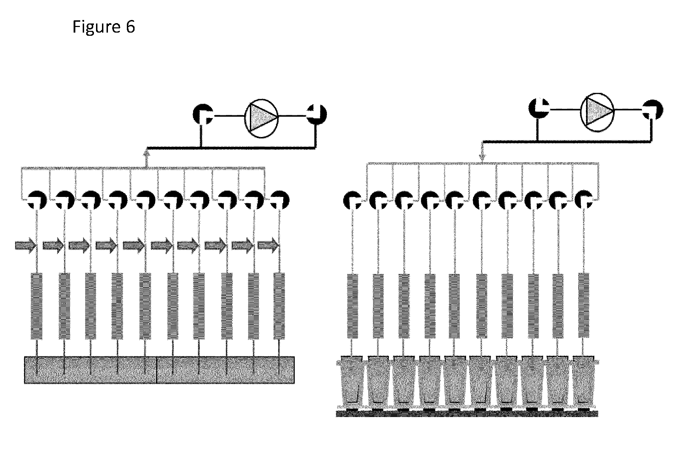

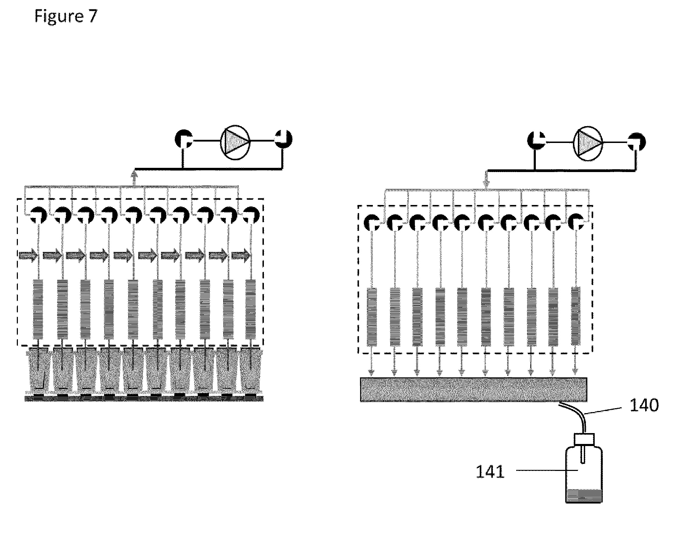

In another embodiment, an automated device for nucleic acid hybridizations and immuno-assays comprises a dispense head 115 consisting of multiple dosing chambers. Controlled delivery of reagents with the dispense head 115, comprising dosing chambers and dispense needles 131, is advantageously compared to the use of conduit and pump reagent delivery. Reagents, such as antibodies and enzymes, are generally expensive and in limited supply. The use of dosing chambers and dispense needles 131 as an assay solution source eliminates dead volume, thereby reducing the waste of valuable reagents. In a preferred embodiment, the dispensing head 115 is controlled in such way that only the exact volume of each assay solution required to carry out the protocol of choice is aspirated from the reagent containers 111 or 113, and the exact same volume of each said assay solution is expelled in the reaction vessel 126 through the dispense needles 131 of the dispense head 115 with little to no waste. The exact volume of each assay solution for each step of the protocol is defined herein as one unit. For clarity, according to the user manual of a diagnostic kit, an assay solution can be used in different steps of the protocol at different volumes, each of which represents one unit of the same assay solution. In a preferred embodiment, Figure 6 and Figure 7 depict aspirating an assay solution from a reagent vessel 111 or 113 into reaction vessels 126 before the incubation step, and aspirating said assay solution from reaction vessels 126 into the waste container 112 after the incubation step, respectively.

In some embodiments, the reagent containers 111 and/or 113 contain appropriate shapes, e.g. slopes, as to avoid waste of assay solutions and fully maximize use of assay solution for assay performance.

In preferred embodiments, the dispense needles 131 are non-disposable and preferably consist of inert material not interfering with assay solutions and sample material.
In some other embodiments, the dispense needles 131 are disposable.

In some embodiments, the dispense head 115 includes a temperature control feature, which heats the assay solutions as required. In some embodiments, the dispense head 115 includes a temperature control feature, which cools the assay solutions as required. In some embodiments, the dispense head 115 includes a temperature control feature, which heats or cools the assay solutions as required. In some embodiments, the dispense head 115 includes a volume control feature, e.g. optical sensor(s), timer(s), pressure sensor(s) for dispensing and aspirating assay solutions.
In some embodiments, the dispense head 115 continuously dispenses and aspirates an assay solution or mixture of assay solutions into and from reaction vessels 126 as an agitating means as required during an incubation step.

In some embodiments, the tray 120 can also include a cooler, such as a ventilated Peltier junction thermoelectric cooler, a fan, or the like. In some embodiments, passive air flow is used for cooling tray 120. In some embodiments, cooling of reaction vessels 126 and/or reagent containers 113 in tray 120 takes place through cavities for cooling 125.

The herein described integrated device has many significant advantages. Automating and controlling the dispensing of assay solutions from dosing chambers eliminates a significant amount of waste that would occur if the assay solutions were pumped into the reaction vessels with a pump and tubing system. Also, all biological samples are processed in parallel, simultaneously and identically within the integrated assay platform.

The dispense head 115 is used to introduce assay solutions into the reaction vessels 126 in parallel for simultaneous introduction of said liquids on the solid supports. A device according to this invention minimizes dead volume, preferably eliminates dead volume of assay solutions. Such device would not involve conduits or pumps, e.g. plastic tubing and peristaltic pumps, for assay solution transfer between reagent containers and reaction vessels.

In some embodiments all reaction trays 114 available in the tray 120 are loaded with a reaction vessel 126 containing a solid support for analyzing a biological sample. Each reaction vessel 126 is visited by a dispense needle 131 and receives assay solutions and is processed according to the protocol of choice. In other embodiments, only some reaction trays 114 in tray 120 are loaded with a reaction vessel 126 containing a solid support for analyzing a biological sample. The dispense head 115 is programmed such that all reaction trays 114 are visited by a dispense needle 131 but only those containing a reaction vessel 126 containing a solid support for analyzing a biological sample receive assay solutions and are processed according to the protocol of choice.

In an embodiment, an automated method of processing an nucleic acid hybridized biological sample comprises: providing at least a nucleic acid hybridization solution in a first reagent container, placing an undeveloped sample into a reaction vessel, contacting the undeveloped sample with the hybridization solution by automatically activating a dispensing head to transfer the hybridization solution from within the first reagent container into the reaction vessel, automatically removing the hybridization solution by the dispensing head from the reaction vessel after the incubation step, transferring from a second reagent container a wash solution into the reaction vessel by the dispensing head, and automatically removing the wash solution from the reaction vessel by the dispensing head after the incubation step. In a further embodiment the hybridization solution in the first reagent container and hybridization solution in the reaction vessel are kept at the same temperature, preferably a temperature optimal for nucleic acid probe - target hybridization, e.g. 49 degrees Celsius. In a further embodiment the first reagent container and reaction vessel are heated by the same heating means.

In an embodiment, an automated method of processing an immuno-stained biological sample comprises: providing at least an antibody solution in a first reagent container, placing an undeveloped sample into a reaction vessel, contacting the undeveloped sample with the antibody solution by automatically activating a dispensing head to transfer the antibody solution from within the first reagent container into the reaction vessel, automatically removing the antibody solution by the dispensing head from the reaction vessel after the incubation step, transferring from a second reagent container a wash solution into the reaction vessel by the dispensing head, and automatically removing the wash solution from the reaction vessel by the dispensing head after the incubation step. In a further embodiment the antibody solution in the first reagent container and antibody solution in the reaction vessel are kept at the same temperature, preferably a temperature optimal for antibody - antigen interaction, e.g. 37 degrees Celsius. In a further embodiment the first reagent container and reaction vessel are heated by the same heating means.

### Examples

The two overviews depicted herein below represent two standard work flows for use of the integrated device according to the present invention for processing multiple biological samples in a nucleic acid hybridization and immuno-assay, respectively.

system workflow for line probe assays
User
Instrument
Take reagents out
of cool storage
Switch ON
Perform self test
Place pre-determined volume of
reagents in reagent containers (113) of
the instrument and select protocol by
control panel (103)
Regulate thermal system to set point
(heat & dissolve both reagents placed
in heated section (113))
Prepare reagents (dilute to pre-
determined volume) and place reagent
containers (111) in instrument
Return unused
reagents to fridge
Prepare samples (denature & incubate)
Load strips and sample into RV (126)
and mark used wells
Load RV into reaction tray (114)
Select number of tests
[START] run by control panel (103)
Execute protocol
Check strips during
first buffer addition
Remove RV (126) from instrument and
remove strips from RV. Analyse result.
Notify user of test completion
clean
Switch OFF

system workflow for line immunoassays

User

Instrument

Take reagents out
of cool storage

Switch ON

Perform self test

Prepare reagents (dilute to pre-
determined volume & warm to room
temp) & place in reagent containers
(111)

Return unused
reagents to fridge

Thermal system remains OFF

Load strips into RV (126) and mark
used wells. Load RV into reaction tray
(114).

Select protocol & number of tests
[START] run by control panel (103)

Add first buffer from a dedicated
reagent container

Pause protocol

Add samples to RV (126) while RV
remains in reagent tray (114)

[CONTINUE] run by control panel (103)

Execute protocol

Notify user of test completion

Remove RV (126) from reagent tray
(114) and remove strips from RV.
Analyse result.

clean

Switch OFF

## Claims

1. An integrated device for processing multiple biological samples, each said sample comprising multiple nucleic acids, proteins or other target molecules or species of molecules, comprising the step of:
dispensing each test solution to effect the binding of nucleic acids, proteins or other target molecules, onto all selected solid supports simultaneously.

2. A device according to claim 1, further comprising an integrated heating block.

3. A device according to claims 1 - 2, further comprising one dispensing unit of test solution.

4. A device according to any of the claims 1 - 3, wherein said selected solid supports are simultaneously processed in parallel.

5. A device according to any of the claims 1 - 4, wherein the dispensing device comprises a multiple channel dosing chamber.

6. A device according to claim 5, wherein the multiple channels are separately operable.

7. A device according to claim 2, wherein the heating block is a metal block.

8. A device according to claim 7, wherein the metal is aluminium.

9. A device according to claim 2, further comprising a tray with reagent vessels and reaction vessels.

10. A device according to claim 9, wherein the reaction vessels are partially covered with a lid.

11. A device according to claims 9 or 10, comprising an edge pivoting means.

12. A device according to any of the claims 1 - 4, wherein the solid support is a strip.

13. A device according to claim 12, wherein the strip is a nitrocellulose, nylon, or a PVDF strip.

14. A device according to claim 12, wherein the solid support is a reaction vessel, preferably positioned vertically.

15. A device according to claim 12 comprising multiple units of reaction vessels.
